# EUROPEAN PATENT SPECIFICATION

(11) **EP 2 334 381 B1**
(45) Date of publication and mention of the grant of the patent: **21.11.2012**
(21) Application number: 09785620.7
(22) Date of filing: 10.09.2009
(51) Int. Cl.: A61Q 9/04, A61K 8/22, A61K 8/38, A61K 8/40

(54) **COMPOSITION AND METHOD FOR REMOVING HAIR FROM THE SKIN**
ZUSAMMENSETZUNG UND VERFAHREN ZUR ENTFERNUNG VON HAAR VON DER HAUT
COMPOSITION ET PROCÉDÉ D'ÉPILATION DE LA PEAU

(30) Priority: 16.09.2008 GB 0816946
(43) Date of publication of application: 22.06.2011
(73) Proprietor: Perachem Limited, Leeds, Yorkshire LS19 7XP (GB)
(72) Inventor: GAUCHÉ, Céline, Fleet, Hampshire GU52 6TF (GB); HAWKES, Jamie, Anthony, Leeds Yorkshire LS20 8FH (GB); LEWIS, David, Malcolm, Otley Yorkshire LS21 2AL (GB)
(74) Representative: Delaney, Jennifer
(86) International application number: PCT/GB2009/051165
(87) International publication number: WO 2010/032035

(56) References cited:
- WO-A1-01/28508
- WO-A1-02/16538
- DE-C- 710 727
- DE-U1- 9 301 342
- US-A- 4 756 845
- US-A- 5 401 497
- US-A1- 2004 083 557
- US-A1- 2006 246 022

## Description

The present invention relates to depilatory compositions and to methods and uses relating thereto.

Hair removal has been practised for a variety of reasons over many centuries and there are many methods by which hair removal can be effected. One such method is chemical depilation in which a chemical is applied to weaken the hair allowing it to be removed more easily. Typically a chemical depilatory composition comprises a material which breaks down the disulfide bonds of keratin in the hair. Most current depilatory compositions are based on thioglycolic acids or salts thereof, for example calcium thioglycolate.

Depilatory compositions of the prior art typically work by reducing the disulfide bonds. However because they contain thioglycolate compounds, these compositions have an unpleasant smell. It is also necessary to use formulations which have a high pH in order for the thioglycolate-based active agent to be effective. The compositions are thus corrosive and can cause serious skin irritation, especially if used on a regular basis.

The present invention seeks to provide an alternative depilatory composition which does not contain a thioglycolic acid derived compound as the only depilatory agent.

According to the present invention there is provided a method of removing hair from the skin, the method comprising the steps of:
a) contacting the skin with a composition comprising a compound of formula (Ia) or (Ib):

   (R-SO₂-OO⁻)ₙMⁿ⁺ (Ia)

   (R-CX-OO⁻)ₙMⁿ⁺ (Ib);

   and
b) removing said composition from the skin along with entrained hair;
wherein X may be O, S or NR¹; n is 1, 2 or 3; M may be hydrogen, an alkali metal, a divalent metal, N(R²)₄, an optionally substituted alkyl or aryl group or a group of formula RC=X;
each of R¹ and R² is selected from hydrogen and an optionally substituted alkyl group; wherein each R is independently selected from a group of formula R³NH, an optionally substituted alkyl, aryl, heteroaryl, cycloalkyl or non-aromatic heterocyclic group, hydroxide, a hydrogen atom, or a group having the formula (II): 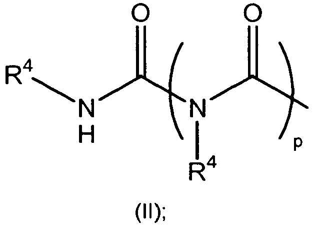 wherein each R⁴ independently represents a hydrogen atom or an alkyl group; p is from 0 to 30 and each R³ is independently selected from an optionally substituted alkyl, aryl, heteroalkyl, cycloalkyl or non-aromatic heterocyclic group, hydroxide, a hydrogen atom or a group of formula (II).

The skilled person will appreciate that the moiety CX represents a carbonyl, thiocarbonyl or imine group.

Suitably when R or R³ is a group having the formula (II), each R⁴ is identical. Most suitably when R or R³ is a group having the formula (II) each R⁴ is a hydrogen atom. Preferably p is 0 to 10, more preferably 0 to 5.

In some preferred embodiments R represents an alkyl group or hydrogen atom. When R is an alkyl group it is preferably an alkyl group having 1 to 20, for example 1 to 14 carbon atoms.

Preferably R³ represents an alkyl group or hydrogen atom. When R³ is an alkyl group it is preferably an alkyl group having 1 to 20, for example 1 to 14 carbon atoms.

The compound of formula (Ib) where R is R³NH and X is O may in some embodiments be a diacyl percarbamate, where M is a further group R³NHCO.

Such diacyl percarbamates are compounds having the formula

R₃-NH-CO-O-O-CO-NH-R³

wherein R³ is as defined above. In such compounds each R³ may be the same or different. Preferred diacyl percarbamates are compounds in which each R³ is an optionally substituted aryl group.

Preferably M is hydrogen, a monovalent cation, for example sodium or potassium, or a divalent cation, for example calcium or magnesium.

In some preferred embodiments X is O and R is R³NH and thus the compound of formula (Ib) is percarbamic acid or a derivative thereof.

Embodiments in which R is R³NH and R³ is hydrogen are especially preferred.

In some preferred embodiments, X is O and R is OH and thus the compound of formula (I) is percarbonic acid or a derivative thereof.

In some preferred embodiments, X is O and R is hydrogen or an optionally substituted alkyl or aryl group and the compound of formula (Ib) is a peracid or a derivative thereof.

In some embodiments the compound of formula (Ia) or (Ib) may be a commercially available stable compound, for example magnesium monoperoxyphthalate.

Preferably however the compound of formula (Ia) or (Ib) is generated *in situ.* By *in situ* we mean that it is generated by formulating a composition comprising precursor components which react to form the compound of formula (Ia) or (Ib).

Suitably the compounds of formula (Ia) or (Ib) are prepared *in situ* by reacting a precursor compound with a peroxide compound.

Suitable precursor compounds for preparing compounds of formula (Ib) include carboxylic acids, carbonic acids, carbamic acids, cyanate salts, amides and ammonium carbonate.

The precursor compound may be selected from known commercially available compounds which react with hydrogen peroxide to form compounds of formula (I). Examples of such compounds include tetraacetylethylenediamine (TAED), sodium nonanoyloxybenzene sulfonate (SNOBS) and dicyandiamide (DiCy). Other compounds of this type are well known to those skilled in the art and are within the scope of the present invention.

Suitable precursor compounds for preparing compounds of formula (Ia) include sulfamic acid salts, sulfamides, sulfonyl esters of formula RSO₂OR' (for example mesylates and tosylates) and sulfonyl thioesters of formula RSO₂SR'.

The peroxide compound may be selected from any source of peroxide known to those skilled in the art. Preferably it is selected from hydrogen peroxide or a hydroperoxide of formula R'OOH wherein R' is a C₁ to C₄ alkyl group.

Alternatively the peroxide compound may comprise a solid peroxide source which releases hydrogen peroxide on contact with water. Such solid peroxide sources include perborates and percarbonates.

Most preferably the peroxide compound is hydrogen peroxide. Preferably this is provided as an aqueous solution of hydrogen peroxide.

In some embodiments hydrogen peroxide may be present in the composition as the perhydroxy anion.

Compounds of general formula (Ib) in which R represents R³NH and R³ is hydrogen may be prepared by the admixture in an aqueous composition of cyanate (for example an alkali metal or ammonium cyanate or a divalent metal cyanate) and hydrogen peroxide (or the perhydroxy anion). The reaction to produce the compound of formula (Ib) is believed to proceed via isocyanic acid HN=C=O.

Such compounds of formula (Ib) in which R represents R³NH and R³ is hydrogen may also be prepared by the admixture in an aqueous composition of formamide and hydrogen peroxide (or the perhydroxy anion). Again, the reaction is believed to proceed via isocyanic acid, as follows:

H-C=(O)-NH₂ + HOOH → HO-C=(O)-NH₂ ⇄ HN=C=O + H₂O

Compounds of formula (Ib) in which R represents R³NH and R³ is hydrogen may also be prepared by the admixture in an aqueous solution of ammonium carbonate and hydrogen peroxide (or the perhydroxy anion). In this case the reaction is believed to proceed via carbamic acid.

Ammonium carbonate is usually provided as an impure compound and sources usually contain significant amounts of ammonium carbamate and ammonium cyanate (NH₄^{+ -}OOC-NH₂ and NH₄^{+ -}OCN), both of which are capable of reaction with hydrogen peroxide to form percarbamic acid:

H-O-O-H + HOOC-NH₂ → H₂N-C=O-O-O-H + -OH

H-O-O-H + HN=C=O → H₂N-C=O-O-O-H

[NH₄⁺⁻OCN + H₂O ⇄ NH=C=O + NH₄OH]

The presence of carbamic acid in ammonium carbonate solution may also be explained due to the reaction of the decomposition products: ammonia and carbon dioxide.

The species present in solution are thus:

NH₄⁺⁻O-(C=O)-OH, O=C=O, NH₃

These may react as follows to form carbamic acid:

O=C=O + NH₃ → HOOC-NH₂

The carbamic acid may then react with hydrogen peroxide to form percarbamic acid.

One preferred method of generating compounds of general formula (Ia) and (Ib) comprises reacting a carbamate compound of the general formula (IIIa) or (IIIb):

R-SO₂-L (IIIa)

R-CX-L (IIIb)

with hydrogen peroxide (or the perhydroxy anion) where R and X are defined above and L is a moiety displaced by the anion ⁻OOH.

Preferably L is selected from hydroxide, phosphonate, phosphinate, thiourea or a quaternary ammonium group; or from groups of the formula -SOₛM' where M' represents a hydrogen atom, or an alkali metal atom, or an ammonium group; or from groups of the formula

- XR⁵ or - Y(R⁵)₂

where X represents an oxygen or sulphur atom, Y represents a nitrogen atom, and R⁵ is selected from hydrogen, optionally substituted alkyl, optionally substituted aryl, optionally substituted amino, cyano, hydroxyl and optionally substituted alkylcarbonyl groups wherein when L is -Y(R⁵)₂ each R⁵ group may be the same or different and is independently selected from the above list, or the groups R⁵ and Y may together represent an optionally substituted heteroaryl or non-aromatic heterocyclic group.

Suitable substituents of an aryl or heteroaryl group include halogen, especially fluorine, chlorine and bromine atoms, and nitro, cyano, hydroxyl, alkyl, haloalkyl, haloalkoxy, alkoxyalkyl, aryloxy, alkoxy, alkoxyalkoxy, amino, mono and di-alkylamino, aminoalkyl, mono- and di-alkylaminoalkyl, amido, mono- and di-alkylamido groups and groups of formula - SO₃M' or -COOM' where M' is as defined above. An especially preferred substituent of an aryl group is -SO₃M'.

Any substituted aryl or heteroaryl group may suitably be substituted by one to three substituents, preferably by one substituent.

Preferred substituents of an alkyl group or of an alkyl moiety within a larger group or of a cycloalkyl or non-aromatic heterocyclic group include halogen, especially fluorine, chlorine or bromine atoms, and nitro, cyano, amidothio (-S-CONH₂), amidoamino (-NH-CONH₂), hydroxyl, alkoxy, haloalkoxy, -COOM', alkoxycarbonyl, amino and mono- and di-alkylamino and -SO₃M' groups, where M' is as defined above.

Preferred substituents of an amino group (including of a larger group such as amido) include alkyl, cyano, hydroxyl, alkoxy, amino, amido, thioamido (-CSNH₂), aminosaccharide, polyaminosaccharide groups (for example glucosamino and polyglucosamino groups). Preferably an amino group is unsubstituted or mono-substituted, or disubstituted by two alkyl, especially methyl, groups.

Preferred heteroaryl or non-aromatic heterocyclic groups contain 1-3 hetero ring atoms selected from oxygen, sulphur or nitrogen. Preferred groups have at least one ring nitrogen atom. Preferred heteroaryl and non-aromatic heterocyclic groups include pyrazine and pyridine groups which are unsubstituted or substituted by a single group selected from - COOM' and -CONH₂. where M' is as defined above; piperidine and morpholine groups which are unsubstituted or substituted by a single C₁₋₄ alkyl group; and bicyclic non-aromatic heterocycles containing one or two nitrogen atoms, for example azabicyclooctane and diazabicyclooctane.

A preferred sub-class of groups L is those of formula - SO₃M' where M' is as defined above.

A preferred sub-class of groups L is those of formula -SX where X represents a cyano group, SO₃M' (where M' is defined above) or an optionally substituted C₁₋₆ alkyl group (preferably unsubstituted or substituted by one to three substituents independently selected from - COOM', -SO₃M', hydroxyl, amidothio, optionally substituted amino (preferably NH₂ or -NH-CONH₂), optionally substituted alkoxy (preferably unsubstituted), optionally substituted amido (preferably -CONH₂) and -COOY, where M' is as defined above and Y is an optionally substituted (preferably unsubstituted) alkyl group).

Especially preferred groups L are -SCN and -SO₃M'. In relation to any of the foregoing definitions, preferably M' represents hydrogen or an alkali metal atom; and especially hydrogen or sodium.

A noteworthy sub-class of groups L is those of formula -OX' where X' represents an optionally substituted (preferably unsubstituted) alkyl group, an optionally substituted (preferably unsubstituted) aryl group or an optionally substituted (preferably unsubstituted) alkylcarbonyl group, or a group -CHO or -OCN. A preferred group L of formula -OX' is -O-PhSO₃M' where M' is defined above.

In an especially preferred embodiment X' is H and L is OH.

A noteworthy sub-class of groups L is those of formula -NX" X'" where X" and X'" may both be alkyl groups or X" may be a hydrogen atom and X'" may be a cyano, alkyl, hydroxyl, amido, amino, aminosaccharide or polyaminosaccharide group.

A noteworthy sub-class of groups L is those of formula -P(=O)R'R" where R' represents hydroxy and R" represents hydrogen or hydroxy or amido.

A noteworthy sub-class Q⁺ of groups L is those connected to the C=O group by an N⁺ atom, the group Q being a quaternary ammonium moiety formed from a tertiary amine, or a heteroaryl or non-aromatic heterocyclic group as detailed above.

Examples of suitable groups L include:
OH , H, imidazoale, N-methyl taurine, nicotinic acid, nicotinic amide, diazabicylcooctane (DABCO), sarcosine, N-methyl amino acids including N-methyl sarcosine and N-methyl glycine, N-methyl morpholine, 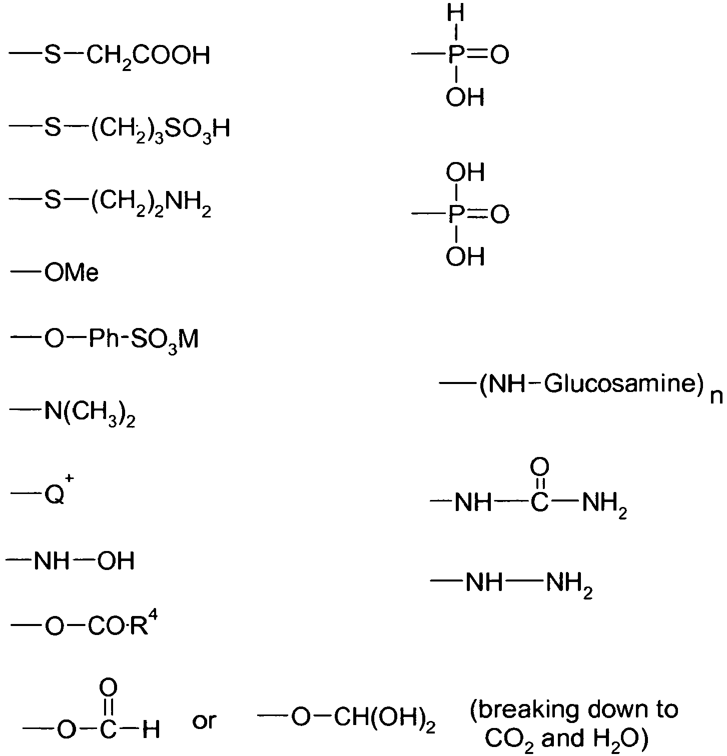 where R⁴ is an alkyl group or a hydrogen atom and, especially:
SO₃M' (especially SO₃H and SO₃Na) 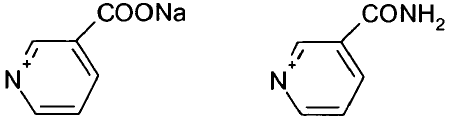 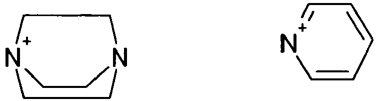
-NHCN

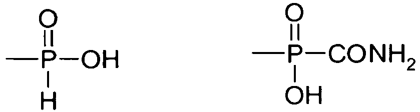

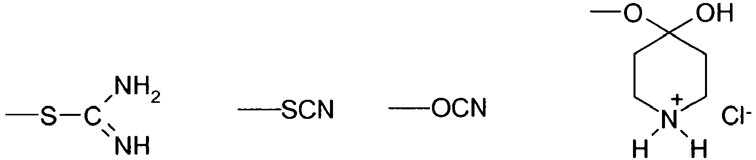

It should be noted that suitable compounds NHR-CO-L include compounds which may have more than one group NHR-CO-, and so many offer more than one point of attack to the peroxyanion. One example of compound of this type is biuret.

Compounds of formula (IIIb) in which R represents hydrogen may be prepared by a reaction employing an alkali metal or ammonium cyanate and a compound HL where L is as defined above. An alkali metal cyanate, notably sodium cyanate, is preferred. The reaction is believed to proceed via isocyanic acid HN=C=O. The resulting compounds (IIIb) may be isolated but if it is wished they may be contacted with hydrogen peroxide, or the perhydroxy anion *in situ* to provide a composition for use in the treatment method of the first aspect of the present invention.

Preferably the process employing an alkali metal or ammonium cyanate and a compound of formula HL is performed in the presence of a solvent, more preferably in aqueous solution. Suitably the pH of the reaction mixture is maintained between 2 and 10, most preferably between 4 and 8. The pH needed will, however, depend on the nature of L.

Where L is a sulphur-containing leaving group the pH is preferably maintained between 4 and 5 for the duration of the reaction.

Where L is a tertiary amine containing leaving group, such as pyridine, the pH is preferably maintained between 6.5 and 7.5 for the duration of the reaction.

Where L contains an alcoholate residue the pH is preferably maintained between 7 and 8 for the duration of the reaction.

Preferably the reaction is carried out at a temperature between 0°C and 100°C, more preferably between 10°C and 50°C, and most preferably at ambient temperature.

In especially preferred embodiments the present invention comprises contacting the skin with a compound of formula (Ib) in which X is O, R is NH₂ and M is an alkali metal, that is a compound of formula (IV):

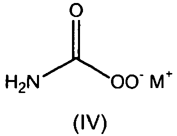

Suitably this compound is generated *in situ* by reaction of a cyanate salt with a peroxide compound. Preferred cyanates salts are alkali metal and ammonium salts. Most preferred are potassium cyanate and especially sodium cyanate.

Step (a) of the method of the present invention preferably comprises contacting the skin with a composition in which the compound of formula (Ia) or (Ib) has been generated *in situ.* Suitably the method thus comprises contacting the skin with a composition comprising a precursor compound and a peroxide compound.

Preferably the precursor compound is present at a concentration of at least 0.1 moldm⁻³, preferably at least 0.25 moldm⁻³, more preferably at least 0.5 moldm⁻³, preferably at least 0.75 moldm⁻³, most preferably at least 1 moldm⁻³.

The precursor compound may be present in a concentration of up to 10 moldm⁻³, for example up to 7.5 moldm⁻³, preferably up to 5 moldm⁻³, more preferably up to 4 moldm⁻³. In some embodiments it may be present in an amount of up to 3.5 moldm⁻³, for example in an amount of from 1.2 moldm⁻³ to 3 moldm⁻³.

In some preferred embodiments the precursor compound is present in an amount of from 2 to 5 moldm⁻³, for example from 3 to 4 moldm⁻³.

Suitably the peroxide compound is present in an amount sufficient to provide the active hydrogen peroxide or the perhydroxy anion at a concentration of at least 0.1 moldm⁻³, more preferably at least 0.2 moldm⁻³, for example at least 0.3 moldm⁻³, preferably at least 0.4 moldm⁻³, more preferably at least 0.5 moldm⁻³.

It may be present in an amount of up to 10 moldm⁻³, for example up to 7.5 moldm⁻³, preferably up to 5 moldm⁻³ and more preferably up to 4 moldm⁻³. It is suitably present in an amount of from 0.6 moldm⁻³ to 3.5 moldm⁻³.

Where there is more than one precursor compound or more than one peroxide compound, the above amounts refer to all such compounds present in the composition.

The skilled person will appreciate that the above amounts refer to the amount of precursor compound and hydrogen peroxide or perhydroxy anion which is added to the composition. Of course once formulated the starting components will suitably react to form the compound of formula (I) *in situ* and thus the final composition may be different.

Suitably the composition is an aqueous composition and preferably comprises from 10-99 wt% water, more preferably from 50-95 wt% water.

The composition may comprise a further solvent or diluent. Suitable solvents and diluents may be selected from those specified for cosmetic use by the Scientific Committee on Consumer Products (SCCP) managed by the Directorate General for Health and Consumer Protection of the European Commission. The SCPP approve a list of chemicals for use which is referred to as the INCL List (International Nomenclature of Cosmetic Ingredients List).

Preferred solvents and diluents include water, ethanol, isopropanol, n-propanol, butanol, propylene glycol, ethylene glycol, monoethyl ether, and mixtures thereof. Other suitable solvents are provided on the INCL list.

In some preferred embodiments the composition comprises a surfactant. This is suitably present in an amount of from 0.001 to 25 wt%, preferably from 0.01 to 20 wt%, more preferably from 0.05 to 15 wt%, for example from 0.1 to 10 wt%.

Suitable surfactants include anionic surfactants, cationic surfactants, non-ionic surfactants and zwitteriomic surfactants. Zwitteriomic surfactants are particularly preferred and most preferred are betaines, for example lauramidopropylbetaine. Other suitable surfactants include those listed on the INCl List. Suitable non-allergic surfactants are known to those skilled in the art.

The composition may comprise one or more thickeners. Preferred thickeners include those provided on the INCl List. Examples of such thickeners include oleic acid, cetyl alcohol, oleyl alcohol, sodium chloride, cetearyl alcohol, stearyl alcohol, synthetic thickeners such as Carbopol, Aculyn and Acrosyl and mixtures thereof. Preferred thickeners for use herein are Aculyn 22 (RTM), steareth-20 methacrylate copolymer; Aculyn 44 (RTM), polyurethane resin and Acusol 830 (RTM), acrylates copolymer which are available from Rohm and Haas, Philadelphia, PA, USA. Additional thickening agents suitable for use herein include sodium alginate or gum arabic, or cellulose derivatives, such as methyl cellulose or the sodium salt of carboxymethylcellulose or some types of acrylic polymers. Thickeners which undergo a change in viscosity with changing pH are particularly preferred. Examples of such thickeners are known to those skilled in the art.

The composition may comprise a mixture of two or more compounds of formula (I).

The composition may comprise one or more further depilatory agents, in addition to the compound or compounds of formula (I). For example, it may comprise a thioglycolic acid derived compound, for example calcium thioglycolate. However in preferred embodiments the composition used in the method of the present invention is substantially free of thioglycolic acid derived compounds such as thioglycolate salts.

The composition may further comprise one or more optional excipients selected from fragrances, stabilisers, antimicrobials, conditioners, emulsion stabilisers, film formers, emulsifiers, antioxidants, chelators, antistatic agents, anti-caking agents, buffers, bulking agents, UV absorbers, moisturising agents, opacifiers, masking agents, reducing agents, humectants, foaming agents. Each of these components may be selected from the INCL list.

The composition suitably has a pH of from 7 to 12, preferably from 8 to 10, more preferably 8.5 to 9.5.

This is lower than the pH of depilatory compositions of the prior art and this is less corrosive to the skin.

The depilatory composition of the present invention is suitably sufficiently mild to enable it to be used on a users face. In particular the method of the present invention may be used for facial hair removal by men instead of shaving. The provision of a depilatory composition which has the efficacy to remove tough male facial hair yet is mild enough to be used on the face is a significant advantage of the composition of the present invention.

Where the method of the present invention is used for removing male facial hair, the composition preferably further comprises a conditioning agent, although a conditioning agent may also be present in other embodiments.

Preferred conditioning agents are compounds of formula: 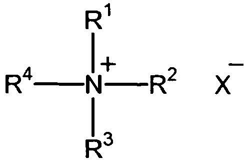 wherein R¹, R², R³ and R⁴ are optionally substituted alkyl groups and X⁻ is a halide. Preferably at least one of R¹, R², R³ and R⁴ is a C₈ to C₂₄ alkyl group.

In some especially preferred embodiments R¹, R² and R³ is each a C₁ to C₄ unsubstituted alkyl group and R⁴ is a C₈ to C₂₄ unsubstituted alkyl group.

Preferably each of R¹, R² and R³ is ethyl or methyl, most preferably methyl.

Preferably R⁴ is a C₁₀ to C₂₀ unsubstituted alkyl group, preferably C₁₂ to C₁₈, for example C₁₄ to C₁₈.

X⁻ is preferably bromide or especially chloride. Preferred conditioning agents are cetyl trimethyl ammonium chloride and cetyl trimethyl ammonium bromide.

The conditioning agent may suitably be present in an amount of from 0.1 to 30wt%, suitably from 0.5 to 20wt%, preferably from 1 to 10wt%.

The composition may be provided in any suitable form. For example, it may be provided as a gel, cream, lotion, aerosol, paste, roll-on or powder.

Suitably it is provided in the form which can be readily spread onto the skin but which holds its shape and position on the skin after application.

In step (a) of the method of the present invention the skin is contacted with the composition for a period sufficient to affect depilation of hair growing thereon. Preferably the skin is contacted with the composition for a period sufficient to effect removal of at least 5% of the hairs growing thereon, preferably at least 10%, more preferably at least 20%, for example at least 30%, preferably at least 40% and most preferably at least 50%. In especially preferred embodiments the composition may remove at least 60% of the hairs growing on the skin in the area treated with the composition, for example at least 70%, preferably at least 80% and most preferably at least 90%. In some embodiments substantially all of the hair, for example greater than 95%, may be removed by the method of the present invention.

Suitably step (a) comprises contacting the skin with the composition for a period of up to 2 hours, for example up to 90 minutes, preferably up to 60 minutes, for example up to 50 minutes, preferably up to 40 minutes and most preferably up to 30 minutes.

Step (a) comprises contacting the composition with the skin for a period of at least 2 seconds, more preferably at least 5 seconds, for example at least 10 seconds, more preferably at least 30 seconds, for example at least 1 minute. It may comprise contacting the skin with the composition for at least 2 minutes, at least 5 minutes, for example at least 7 minutes or at least 10 minutes.

The composition applied to the skin may have a temperature of from 0 to 50°C, for example 5 to 40°C, preferably 10 to 30°C, preferably 15 to 25°C. It suitably is applied at ambient temperature.

In step (b) the composition and entrained hair may be removed from the skin by any suitable means. It may for example be simply rinsed away with water, or it may be scraped off, for example using a spatula. It may be rubbed off using the hands or it may be wiped clean with a cloth.

It is believed that in the method of the present invention the depilatory action is achieved by oxidative cleavage of the cystine disulfide bonds of the keratin in the hair. This involves a very different mechanism to compositions of the prior art in which disulfide bonds are reduced. The compound of formula (Ia) or (Ib) used in the present invention is an oxidant. Preferably the composition is substantially free of reducing agents.

The present invention may involve the use of a depilatory product comprising a precursor compound and a peroxide compound wherein the precursor compound is selected from a compound of formula (IIIa) or (IIIb), a carboxylic acid or a derivative thereof, carbonic acid or a derivative thereof, a carbamic acid or a derivative thereof, a cyanate salt, amide or ammonium carbonate; and the peroxide compound is a compound selected from hydrogen peroxide or a compound which may be activated to provide hydrogen peroxide or the perhydroxy anion.

Preferred precursor compounds and peroxide compounds are as defined herein. In preferred embodiments the precursor compound is selected from ammonium carbonate, ammonium carbamate, a divalent metal cyanate; a monovalent metal cyanate and mixtures thereof.

The depilatory product may comprise two compositions for admixture immediately prior to application.

Suitably the depilatory product may comprise a first composition comprising a precursor compound and a second composition comprising a peroxide compound.

In some preferred embodiments the first composition comprises one or more alkali metal cyanate salts. In some preferred embodiments the second composition comprises hydrogen peroxide.

Further ingredients previously mentioned herein may be provided in either or each of the first and second compositions. The first and second compositions may be liquid compositions which undergo a change in viscosity upon admixture.

Preferably any such change in viscosity is due to a change in pH upon admixture of the compositions.

In some alternative embodiments the depilatory product may comprise a single precursor composition which may be activated to form an activated depilatory composition. Such a precursor composition may comprise for example, a stabilised peroxide compound which only reacts with the precursor compound upon for example, exposure to air agitation or application of a force, for example a shear force.

The precursor compound and/or the peroxide compound may be encapsulated so that the two do not react in the single precursor composition during storage. The composition may be such that when a shear force is applied, for example as the composition is expelled from the container (for example through a nozzle or aerosol delivery mechanism) the two precursor compounds and the peroxide compound are then able to react to form the compound of formula (I), which is the active depilatory agent.

The precursor compound and peroxide compound may alternatively be held in separate phases of an emulsion. The emulsion may be induced to break down by application of a shear force during delivery of the composition to the skin thus forming the active depilatory agent *in situ.*

In an alternative embodiment, the depilatory product may comprise a solid composition comprising a solid precursor compound and a solid peroxide compound. The active agent of formula (I) may be generated as desired prior to application of the composition to the skin by addition of a diluent.

Suitable diluents include any cosmetically acceptable diluent or carrier as described above. In such embodiments the cosmetic product may comprise a separate liquid composition in addition to the solid composition; the liquid composition comprising a diluent or diluent mixture for addition to the solid composition prior to application to the skin.

In some especially preferred embodiments the depilatory product comprises a first solid composition comprising one or more precursor compounds, for example one or more alkali metal cyanates and a second liquid composition comprising a peroxide compound, for example hydrogen peroxide.

As mentioned above, either of the first and/or second compositions may comprise one or more further excipients. A preferred thickener for use in a solid composition is hydroxymethyl cellulose.

A method of preparing a depilatory product as defined herein may comprise admixing a first composition comprising a precursor compound and a second composition comprising a peroxide compound.

In some embodiments the depilatory product may be provided in a bicompartment container in which the first composition is held in a first compartment and the second composition is held in a second compartment, of the same container. Preferably the bicompartment container is arranged to deliver the first and second compositions to the same locus. This may be achieved by providing adjacent outlets from the first and second compartments. Alternatively, the first and second compartments may be arranged to deliver the first and second precursor compositions into a common passageway in which they are contacted prior to exiting the container through a single outlet. Bicompartment containers of this type are known to the person skilled in the art. One such example is a squeezable tube (known as a "dual tube") having two compartments comprising the two precursor compositions. Squeezing the tube causes the two compositions to be delivered through adjacent outlets such that they come into immediate contact with each other on exiting the container. Other embodiments of bicompartment containers also include bottles or canisters for holding mousses, gels or sprays which are provided with a single actuator which effects delivery of the two precursor compositions to the same locus via the same or adjacent outlets. Alternatively the depilatory product of the present invention may be provided as two discrete compositions which are packaged separately in individual containers.

The present invention may involve the use of a kit comprising a composition or a depilatory product as defined herein and instructions for hair removal.

Where the depilatory product comprises two precursor compositions, the kit may further comprise instruction for preparing the active depilatory composition prior to application. The composition may suitably be prepared immediately prior to application. The active depilatory composition may be prepared up to 12 hours prior to application, for example up to 8 hours, preferably up to 6 hours, preferably up to 4 hours, for example up to 2 hours, preferably up to 1 hour, for example up to 30 minutes and most preferably up to 10 minutes.

In embodiments in which the depilatory product comprises a solid composition which must be mixed with a diluent prior to admixture, the kit may further comprise instructions for preparing the active depilatory composition. It may optionally comprise a container and/or stirrer for assisting in the preparation of said active composition.

The kit further may comprise a tool for assisting hair removal. Such tools include for example a cloth or a plastic spatula and are well known to those skilled in the art.

The kit may further comprise a conditioning agent for application to the skin following hair removal. The conditioning agent may be provided in a lotion or impregnated on a wipe.

The present invention will now be further described with reference to the following non-limiting examples.

### Example 1

| **Part 1** | | **Part 2** | |
|---|---|---|---|
| 100% | NaOCN | 49.38% | H₂O₂ (12% Soln) |
| | | 49.38% | Water |
| | | 1.24% | Laurylamidopropylbetaine |

- Prepare hair treatment mixture, by mixing 1 gram of Part 1 and 9 grams of Part 2.
- pH of prepared mixture is pH9.
- Dip 1 gram of hair into mixture and leave at room temperature for 15 minutes.
- Rinse with cold or warm water.

The hair appeared to disintegrate to form a paste of proteinous material. It was discoloured and swelled up to five times its original volume. The hair broke with very little force - simply by rinsing with water.

### Example 2

| **Part 1** | | **Part 2** | |
|---|---|---|---|
| 100% | NaOCN | 29.41% | H₂O₂(12% Soln) |
| | | 69.41% | Water |
| | | 1.18% | Laurylamidopropylbetaine |

- Prepare hair treatment mixture, by mixing 1.5 grams of Part 1 and 8.5 grams of Part 2.
- pH of prepared mixture is pH9.3
- Dip 1 gram of hair into mixture and leave at room temperature for 30 minutes.
- Rinse with cold or warm water.

As in example 1, the hair was visibly damaged to an extent that indicated that this composition would be suitable to remove hair from the skin.

### Example 3

| **Part 1** | | **Part 2** | |
|---|---|---|---|
| 100% | NaOCN | 74.44% | H₂O₂ (12% Soln) |
| | | 24.44% | Water |
| | | 1.12% | Laurylamidopropylbetaine |

- Prepare hair treatment mixture, by mixing 1 gram of Part 1 and 9 grams of Part 2.
- pH of prepared mixture is pH9.4
- Dip 1 gram of hair into mixture and leave at room temperature for 5 minutes.
- Rinse with cold or warm water.

As in example 1, the hair was visibly damaged to an extent that indicated that this composition would be suitable to remove hair from the skin.

### Example 4

| **Part 1** | | **Part 2** | |
|---|---|---|---|
| 100% | KOCN | 49.38% | H₂O₂ (12% Soln) |
| | | 49.38% | Water |
| | | 1.24% | Laurylamidopropylbetaine |

- Prepare hair treatment mixture, by mixing 1 gram of Part 1 and 9 grams of Part 2.
- pH of prepared mixture is pH9.7
- Dip 1 gram of hair into mixture and leave at room temperature for 15 minutes.
- Rinse with cold or warm water.

As in example 1, the hair was visibly damaged to an extent that indicated that this composition would be suitable to remove hair from the skin.

### Example 5

| **Part 1** | | **Part 2** | |
|---|---|---|---|
| 100% | NaOCN | 49.32% | H₂O₂ (12% Soln) |
| | | 49.32% | Water |
| | | 1.23% | Laurylamidopropylbetaine |
| | | 0.13% | Acetic Acid |

- Prepare hair treatment mixture, by mixing 1 gram of Part 1 and 7 grams of Part 2.
- pH of prepared mixture is pH8
- Dip 1 gram of hair into mixture and leave at room temperature for 15 minutes.
- Rinse with cold or warm water.

As in example 1, the hair was visibly damaged to an extent that indicated that this composition would be suitable to remove hair from the skin.

### Example 6

| **Part 1** | | **Part 2** | |
|---|---|---|---|
| 100% | NaOCN | 49.08% | H₂O₂ (12% Soln) |
| | | 49.08% | Water |
| | | 1.23% | Laurylamidopropylbetaine |
| | | 0.61% | 2-amino-2-methyl-1-propanol |

- Prepare hair treatment mixture, by mixing 1 gram of Part 1 and 7 grams of Part 2.
- pH of prepared mixture is pH11
- Dip 1 gram of hair into mixture and leave at room temperature for 15 minutes.
- Rinse with cold or warm water.

As in example 1, the hair was visibly damaged to an extent that indicated that this composition would be suitable to remove hair from the skin.

### Example 7

A depilatory product was prepared comprising a powder composition as part 1 and a liquid composition as part 2 as follows:

| **Part 1 -** | **Depilatory Powder** | |
|---|---|---|
| | Quantity in Part | Quantity in final mixture |
| Sodium cyanate | 51.7% | 15% |
| Potassium cyanate | 34.5% | 10% |
| Hydroxyethylcellulose | 13.8% | 4% |

| **Part 2 -** | **Depilatory Solution** | |
|---|---|---|
| | Quantity in Part | Quantity in final mixture |
| Hydrogen peroxide 12% solution | 46.9% | 33.3% |
| Water | 47.5% | 33.7% |
| Cetyltrimethylammonium chloride, 25 wt.% solution in water | 5.6% | 4% |

2.9g of Depilatory Powder was mixed with 7.1g of Depilatory Solution and stirred for 10 to 20 seconds to obtain a paste. This was applied to the leg of a user straight away and allowed to remain on the skin for 5 to 10 min before rinsing with warm water. On the area of skin thus treated substantially all of the hair had been removed. No skin irritation was observed.

### Example 8

A depilatory product was prepared comprising a powder composition as part 1 and a liquid composition as part 2 as follows:

| **Part 1** | **Depilatory Powder** | |
|---|---|---|
| | Quantity in Part | Quantity in final mixture |
| Potassium cyanate | 83.3% | 20% |
| Hydroxyethylcellulose | 16.7% | 4% |

| **Part 2** | **Depilatory Solution** | |
|---|---|---|
| | Quantity in Part | Quantity in final mixture |
| Hydrogen peroxide 12% solution | 43.8% | 33.3% |
| Water | 50.9% | 38.7% |
| Cetyltrimethylammonium chloride, 25 wt.% solution in water | 5.3% | 4% |

2.4g of Depilatory Powder was mixed with 7.6g of Depilatory Solution and stirred for 10 to 20 seconds to obtain a paste. This was applied straight away to the leg of a user for 5 to 10 minutes before rinsing with warm water. On the area of the skin thus treated substantially all of the hair had been removed. No skin irritation was observed.

## Claims

1. A method of removing hair from the skin, the method comprising the steps of:
a) contacting the skin with a composition comprising a compound of formula (Ia) or (Ib):
(R-SO₂-OO⁻)ₙMⁿ⁺ (Ia)
(R-CX-OO⁻)ₙMⁿ⁺, (Ib) ;
and
b) removing said composition from the skin along with entrained hair;
wherein X may be O, S or NR¹, n is 1, 2 or 3, M may be hydrogen, an alkali metal, a divalent metal, N(R²)₄, an optionally substituted alkyl or aryl group or a group of formula RC=X;
each of R¹ and R² is selected from hydrogen and an optionally substituted alkyl group; wherein each R is independently selected from a group of formula R³NH, an optionally substituted alkyl, aryl, heteroaryl, cycloalkyl or non-aromatic heterocyclic group, hydroxide, a hydrogen atom, or a group having the formula (II): 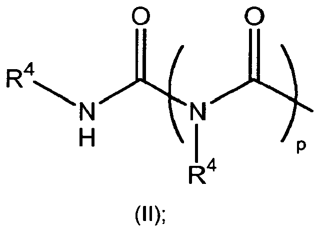 wherein each R⁴ independently represents a hydrogen atom or an alkyl group; p is from 0 to 30 and each R³ is independently selected from an optionally substituted alkyl, aryl, heteroalkyl, cycloalkyl or non-aromatic heterocyclic group, hydroxide, a hydrogen atom or a group of formula (II).

2. A method according to claim 1 wherein in the composition used in step (a) the compound of formula (Ia) or (Ib) is generated *in situ* by the reaction of a precursor compound with a peroxide compound.

3. A method according to claim 2 wherein the precursor compound is selected from a carboxylic acid or a derivate thereof; carbonic acid or a derivate thereof; a carbamic acid or a derivate thereof; a cyanate salt, amide or ammonium carbonate or a compound of formula (IIIa) or (IIIb):
R-SO₂-L (IIIa)
R-CX-L (IIIb)
wherein L is a moiety displaced by the anion ⁻OOH; and the peroxide compound is a compound selected from hydrogen peroxide or a compound which may be activated to provide hydrogen peroxide or the perhydroxy anion.

4. A method according to claim 2 or claim 3 wherein the precursor compound is selected from a carboxylic acid, carbonic acid, carbamic acid, a carbonate salt, a cyanate salt, amides and ammonium carbonate.

5. A method according to claim 4 wherein the precursor compound is selected from a cyanate salt, carbonic acid or ammonium carbonate.

6. A method according to any of claims 2 to 5 wherein the peroxide compound is hydrogen peroxide.

7. A method according to any preceding claim wherein the composition contacted with the skin in step (a) is prepared from a first composition comprising a precursor compound and a second composition comprising a peroxide compound.

8. A method according to claim 7 wherein the first composition comprises one or more of ammonium carbonate, ammonium carbamate, a divalent metal cyanate and a monovalent metal cyanate.

9. A method according to claim 8 wherein the first composition comprises sodium cyanate, potassium cyanate or a mixture thereof.

10. A method according to any preceding claim wherein the composition contacted with the skin in step (a) has a pH of from 8 to 10.

11. A method according to any preceding claim for the removal of male facial hair.

## Patentansprüche

1. Verfahren zum Entfernen von Haar von der Haut, wobei das Verfahren die folgenden Schritte umfasst:
a) Inkontaktbringen der Haut mit einer Zusammensetzung, umfassend eine Verbindung der Formel (Ia) oder (Ib):
(R-S0₂-OO⁻)ₙMⁿ⁺ (Ia)
(R-CX-OO⁻)ₙMⁿ⁺ (Ib);
und
b) Entfernen der Zusammensetzung von der Haut zusammen mit dem mitgeführten Haar;
wobei X O, S oder NR¹ sein kann, n 1, 2 oder 3 ist, M Wasserstoff, ein Alkalimetall, ein zweiwertiges Metall, N(R2)₄, eine gegebenenfalls substituierte Alkyl- oder Arylgruppe oder eine Gruppe der Formel RC=X sein kann;
jedes von R¹ und R² ausgewählt ist aus Wasserstoff und einer gegebenenfalls substituierten Alkylgruppe;
wobei jedes R unabhängig ausgewählt ist aus einer Gruppe der Formel R³NH, einer gegebenenfalls substituierten Alkyl-, Aryl-, Heteroaryl-, Cycloalkylgruppe oder einer nicht aromatischen heterocyclischen Gruppe, einem Hydroxid, einem Wasserstoffatom oder einer Gruppe der Formel (II): 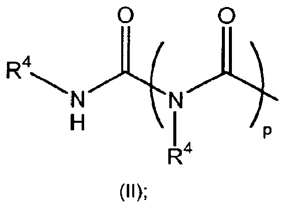 wobei jedes R⁴ unabhängig für ein Wasserstoffatom oder eine Alkylgruppe steht; p gleich 0 bis 30 ist und jedes R³ unabhängig ausgewählt ist aus einer gegebenenfalls substituierten Alkyl-, Aryl-, Heteroalkyl-, Cycloalkylgruppe oder einer nicht aromatischen heterocyclischen Gruppe, einem Hydroxid, einem Wasserstoffatom oder einer Gruppe der Formel (II).

2. Verfahren nach Anspruch 1, wobei in der in Schritt (a) verwendeten Zusammensetzung die Verbindung der Formel (Ia) oder (Ib) *in situ* durch die Reaktion einer Vorläuferverbindung mit einer Peroxidverbindung erzeugt wird.

3. Verfahren nach Anspruch 2, wobei die Vorläuferverbindung ausgewählt ist aus einer Carbonsäure oder einem Derivat davon; einer Kohlensäure oder einem Derivat davon; einer Carbaminsäure oder einem Derivat davon; einem Cyanatsalz, Amid oder Ammoniumcarbonat oder einer Verbindung der Formel (IIIa) oder (IIIb):
R-SO₂-L (IIIa)
R-CX-L (IIIb)
wobei L eine durch das Anion ⁻OOH verschobene Komponente ist; und die Peroxidverbindung eine Verbindung ist, ausgewählt aus Wasserstoffperoxid, oder einer Verbindung, die aktiviert werden kann, um Wasserstoffperoxid oder das Perhydroxyanion bereitzustellen.

4. Verfahren nach Anspruch 2 oder Anspruch 3, wobei die Vorläuferverbindung ausgewählt ist aus einer Carbonsäure, Kohlensäure, Carbaminsäure, einem Carbonatsalz, einem Cyanatsalz, Amiden und Ammoniumcarbonat.

5. Verfahren nach Anspruch 4, wobei die Vorläuferverbindung ausgewählt ist aus einem Cyanatsalz, Kohlensäure oder Ammoniumcarbonat.

6. Verfahren nach einem der Ansprüche 2 bis 5, wobei die Peroxidverbindung Wasserstoffperoxid ist.

7. Verfahren nach einem der vorhergehenden Ansprüche, wobei die Zusammensetzung, die in Schritt (a) mit der Haut in Kontakt gebracht wird, aus einer ersten Zusammensetzung, umfassend eine Vorläuferverbindung, und einer zweiten Zusammensetzung, umfassend eine Peroxidverbindung, hergestellt wird.

8. Verfahren nach Anspruch 7, wobei die erste Zusammensetzung eines oder mehrere aus Ammoniumcarbonat, Ammoniumcarbamat, einem zweiwertigen Metallcyanat und einem einwertigen Metallcyanat umfasst.

9. Verfahren nach Anspruch 8, wobei die erste Zusammensetzung Natriumcyanat, Kaliumcyanat oder ein Gemisch davon umfasst.

10. Verfahren nach einem der vorhergehenden Ansprüche, wobei die Zusammensetzung, die in Schritt (a) mit der Haut in Kontakt gebracht wird, einen pH-Wert von 8 bis 10 aufweist.

11. Verfahren nach einem der vorhergehenden Ansprüche zum Entfernen von männlichem Gesichtshaar.

## Revendications

1. Procédé d'épilation de la peau, le procédé comprenant les étapes consistant à :
a) mettre la peau en contact avec une composition comprenant un composé de formule (Ia) ou (Ib) :
(R-SO2-OO⁻)ₙMⁿ⁺ (Ia)
(R-CX-OO⁻)ₙMⁿ⁺ (Ib)
et
b) enlever ladite composition de la peau ainsi que le poil entrainé ;
dans lesquelles X peut être 0, S ou NR¹, n est 1, 2 ou 3, M peut être hydrogène, un métal alcalin, un métal divalent, N (R²) ₄, un groupement alkyle ou aryle éventuellement substitué ou un groupement de formule RC=X ; chacun de R¹ et R² est choisi parmi hydrogène et un groupement alkyle éventuellement substitué ;
dans lesquelles chaque R est choisi indépendamment parmi un groupement de formule R³NH, un groupement alkyle, aryle, hétéroaryle, cycloalkyle ou hétérocyclique non aromatique éventuellement substitué, hydroxyde, un atome d'hydrogène, ou un groupement ayant la formule (II) : 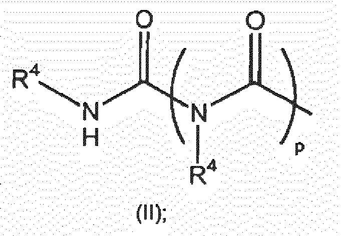 dans laquelle chaque R⁴ représente indépendamment un atome d'hydrogène ou un groupement alkyle ; p est de 0 à 30 et chaque R³ est choisi indépendamment parmi un groupement alkyle, aryle, hétéroalkyle, cycloalkyle ou hétérocyclique non aromatique éventuellement substitué, hydroxyde, un atome d'hydrogène ou un groupement de formule (II).

2. Procédé selon la revendication 1, **caractérisé en ce que** dans la composition utilisée à l'étape a), le composé de formule (Ia) ou (Ib) est généré *in situ* par la réaction d'un composé précurseur avec un composé peroxyde.

3. Procédé selon la revendication 2, **caractérisé en ce que** le composé précurseur est choisi parmi un acide carboxylique ou un dérivé de celui-ci ; un acide carbonique ou un dérivé de celui-ci ; un acide carbamique ou un dérivé de celui-ci ; un sel de cyanate, un amide ou un carbonate d'ammonium ou un composé de formule (IIIa) ou (IIIb) :
R-SO₂-L (IIIa)
R-CX-L (IIIb)
dans lesquelles L est un motif déplacé par l'anion OOH ; et le composé peroxyde est un composé choisi parmi le peroxyde d'hydrogène ou un composé qui peut être activé pour donner le peroxyde d'hydrogène ou l'anion perhydroxy.

4. Procédé selon la revendication 2 ou la revendication 3, **caractérisé en ce que** le composé précurseur est choisi parmi un acide carboxylique, un acide carbonique, un acide carbamique, un sel de carbonate, un sel de cyanate, des amides et le carbonate d'ammonium.

5. Procédé selon la revendication 4, **caractérisé en ce que** le composé précurseur est choisi parmi un sel de cyanate, l'acide carbonique ou le carbonate d'ammonium.

6. Procédé selon l'une quelconque des revendications 2 à 5, **caractérisé en ce que** le composé peroxyde est le peroxyde d'hydrogène.

7. Procédé selon l'une quelconque des revendications précédentes, **caractérisé en ce que** la composition mise en contact avec la peau à l'étape (a) est préparée à partir d'une première composition comprenant un composé précurseur et une deuxième composition comprenant un composé peroxyde.

8. Procédé selon la revendication 7, **caractérisé en ce que** la première composition comprend un ou plusieurs parmi le carbonate d'ammonium, le carbamate d'ammonium, un cyanate de métal divalent et un cyanate de métal monovalent.

9. Procédé selon la revendication 8, **caractérisé en ce que** la première composition comprend le cyanate de sodium, le cyanate de potassium ou un mélange de ceux-ci.

10. Procédé selon l'une quelconque des revendications précédentes, **caractérisé en ce que** la composition mise en contact avec la peau à l'étape (a) a un pH de 8 à 10.

11. Procédé selon l'une quelconque des revendications précédentes, destiné à l'épilation du visage chez l'homme.
